# EUROPEAN PATENT APPLICATION

(11) **EP 4 495 109 A1**
(43) Date of publication of application: **22.01.2025**
(21) Application number: 23769902.0
(22) Date of filing: 17.03.2023
(51) Int. Cl.: C07D 401/12, C07D 213/63, C07D 213/75, A61K 31/444, A61K 31/496, A61P 35/00

(54) **SALT OF SUBSTITUTED AMINO SIX-MEMBERED NITRIC HETEROCYCLIC COMPOUND, CRYSTAL FORM THEREOF, METHOD FOR PREPARING SAME, AND USE THEREOF**

(30) Priority: 18.03.2022 CN 202210268968
(71) Applicant: Shanghai Runshi Medical Technology Co., Ltd, Shanghai 200040 (CN); Shanghai Institute of Materia Medica, Chinese Academy of Sciences, Shanghai 201203 (CN)
(72) Inventor: ZHANG, Ao, Shanghai 201203 (CN); LI, Zhihuai, Shanghai 200040 (CN); HAO, Hongru, Shanghai 200040 (CN); GENG, Meiyu, Shanghai 201203 (CN); CAO, Zefeng, Shanghai 200040 (CN); SONG, Zilan, Shanghai 201203 (CN); HE, Ying, Shanghai 200040 (CN); YIN, Lu, Shanghai 200040 (CN)
(74) Representative: Gille Hrabal Partnerschaftsgesellschaft mbB Patentanwälte
(86) International application number: PCT/CN2023/082140
(87) International publication number: WO 2023/174400

(57) **Abstract**

The present invention provides a salt of a substituted amino six-membered nitric heterocyclic compound, a crystal form thereof, a method for preparing same, and use thereof. Specifically provided is a dihydrochloride salt of a compound represented by formula (I). The salt can be prepared as a crystal form, the crystal form preferably having the following advantages: good solubility, low hygroscopicity, stable quality, and crystal form stability, thus being more easily developed into drugs as compared with the compound represented by formula (I) or other salts.

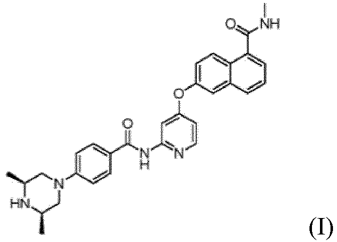

## Description

The present application claims priority to the prior application with the patent application No. 202210268968.7 entitled "SALT OF SUBSTITUTED AMINO SIX-MEMBERED NITRIC HETEROCYCLIC COMPOUND, CRYSTAL FORM THEREOF, METHOD FOR PREPARING SAME, AND USE THEREOF" and filed with China National Intellectual Property Administration on Mar. 18, 2022, which is incorporated herein by reference in its entirety.

### TECHNICAL FIELD

The present disclosure belongs to the field of pharmaceutical chemistry, and particularly relates to a salt of a substituted amino six-membered nitrogen heterocyclic compound, a crystal form thereof, a preparation method therefor, and use thereof.

### BACKGROUND

Fibroblast growth factor receptors (FGFRs) are important molecular typing targets of tumors, and are abnormally activated in various tumors, especially refractory tumors and Chinese characteristic tumor species. Currently, most of clinical studies on inhibitors targeting FGFR are in an early stage, and the FGFR inhibitors in clinical research are mainly multi-target, have weak inhibitory activity on FGFR, and often enhance antagonistic effects on human kinase insert domain containing receptors (KDRs), causing serious adverse effects, thus greatly limiting the application of the anti-tumor effects of the inhibitors targeting FGFR and the maximization of the clinical efficacy. However, FGFR and KDR have the ability to compensate for activation, suggesting that they compensate for each other to mediate drug resistance. In fact, it has been reported that the activation of FGFR is a drug resistance mechanism of KDR inhibitors, and in addition, the role of the KDR family in strengthening immunosuppression and promoting tumors has been gradually revealed in recent years. Therefore, the development of inhibitors co-targeting FGFR and KDR has important clinical application potential.

It should not be ignored that in recent years, tumor therapy is not limited to tumor cells themselves, and the role of the inseparable tumor microenvironment, as a functional whole, in promoting tumor progression and mediating drug resistance attracts great attention. Among them, tumor-associated macrophages (TAMs) are important microenvironment stromal cells, which not only directly inhibit effector T cells from exerting killing effects and synergistically promote tumor immunosuppressive agent microenvironment, but also promote the growth and metastasis of tumor cells and mediate drug resistance in tumors by promoting angiogenesis in tumors through multiple processes. Colony stimulating factor-1 receptor (CSF-1R) is expressed in macrophages and is crucial for maintenance of TAM differentiation towards M2 polarized phenotype, and its proliferation and survival. Based on this, synchronously targeting FGFR/KDR/CSF-1R can not only antagonize tumor cells themselves, but also regulate the tumor microenvironment, thereby being beneficial to antagonizing tumors through multiple processes, remodeling the immunosuppressive microenvironment of the organism, and slowing the occurrence of acquired drug resistance.

A compound of formula (I) is a novel FGFR/CSF1R/KDR-targeting small molecule inhibitor developed based on the above mechanism, and pre-clinical pharmacological studies show that the compound has relatively good efficacy in various tumor models.

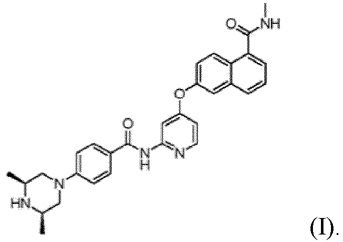

The PCT patent document WO2017/140269A1 discloses a structure of the compound of formula (I), a preparation method therefor, and pharmaceutical use thereof in preventing and/or treating diseases associated with FGFR.

The PCT patent document WO2021/170078A1 discloses use of the compound of formula (I) as a CSF-1R inhibitor.

None of the patent documents described above have investigated the specific salt forms of the compound of formula (I), crystal forms thereof, and preparation thereof.

### SUMMARY

The compound of formula (I) is a brand new compound drug molecule, and through systematic research on the salt forms and the crystal forms of the compound, the inventors unexpectedly found a pharmaceutical form that is most druggable.

The inventors found that the compound of formula (I) obtained according to the prior art has poor druggability due to its own physicochemical properties, etc., for example, the solubility of the compound of formula (I) is relatively low, etc. Therefore, further research on pharmaceutical forms of the compound of formula (I) is necessary to meet the superior pharmaceutical requirements.

In order to solve the problems described above, the present disclosure provides a dihydrochloride salt of a compound of formula (I), and the dihydrochloride salt of the compound of formula (I) has a structure of formula (II):

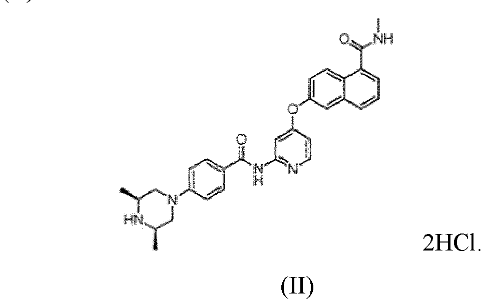

According to an embodiment of the present disclosure, the dihydrochloride salt of the compound of formula (I) is in a solid form.

According to an embodiment of the present disclosure, the dihydrochloride salt of the compound of formula (I) is in a crystalline form.

The present disclosure further provides crystal form I of the dihydrochloride salt of the compound of formula (I), which has characteristic peaks at 2θ angles of 5.0±0.2°, 13.5±0.2°, 19.3±0.2°, 20.5±0.2°, 21.4±0.2°, and 25.2±0.2° by X-ray powder diffraction using Cu-Kα radiation.

According to an embodiment of the present disclosure, the crystal form I of the dihydrochloride salt of the compound of formula (I) has characteristic peaks at 2θ angles of 5.0±0.2°, 10.4±0.2°, 13.5±0.2°, 19.3±0.2°, 20.5±0.2°, 21.4±0.2°, 23.4±0.2°, and 25.2±0.2° by X-ray powder diffraction using Cu-Kα radiation.

According to an embodiment of the present disclosure, the crystal form I of the dihydrochloride salt of the compound of formula (I) has characteristic peaks at 2θ angles of 5.0±0.2°, 10.4±0.2°, 13.5±0.2°, 19.3±0.2°, 20.5±0.2°, 21.4±0.2°, 22.5±0.2°, 23.4±0.2°, 23.9±0.2°, 24.3±0.2°, and 25.2±0.2° by X-ray powder diffraction using Cu-Kα radiation.

According to an embodiment of the present disclosure, the crystal form I of the dihydrochloride salt of the compound of formula (I) has characteristic peaks at 2θ angles of 5.0±0.2°, 10.4±0.2°, 11.2±0.2°, 13.5±0.2°, 19.3±0.2°, 20.5±0.2°, 21.4±0.2°, 22.0±0.2°, 22.5±0.2°, 23.4±0.2°, 23.9±0.2°, 24.3±0.2°, 25.2±0.2°, and 26.6±0.2° by X-ray powder diffraction using Cu-Kα radiation.

According to an embodiment of the present disclosure, the crystal form I of the dihydrochloride salt of the compound of formula (I) has characteristic peaks at 2θ angles of 5.0±0.2°, 10.0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, and 30.1±0.2° by X-ray powder diffraction using Cu-Kα radiation.

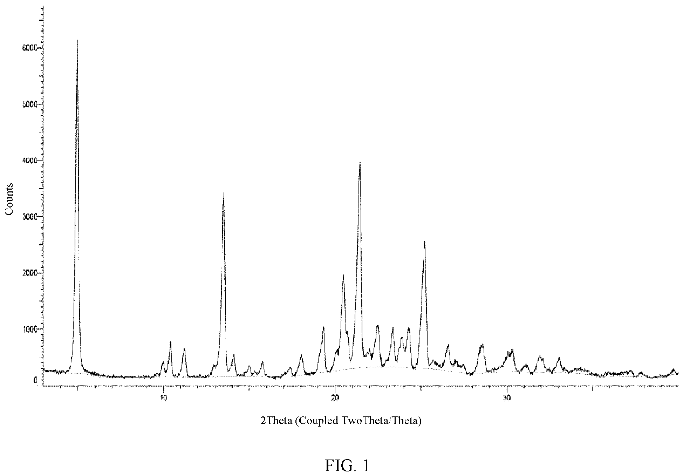

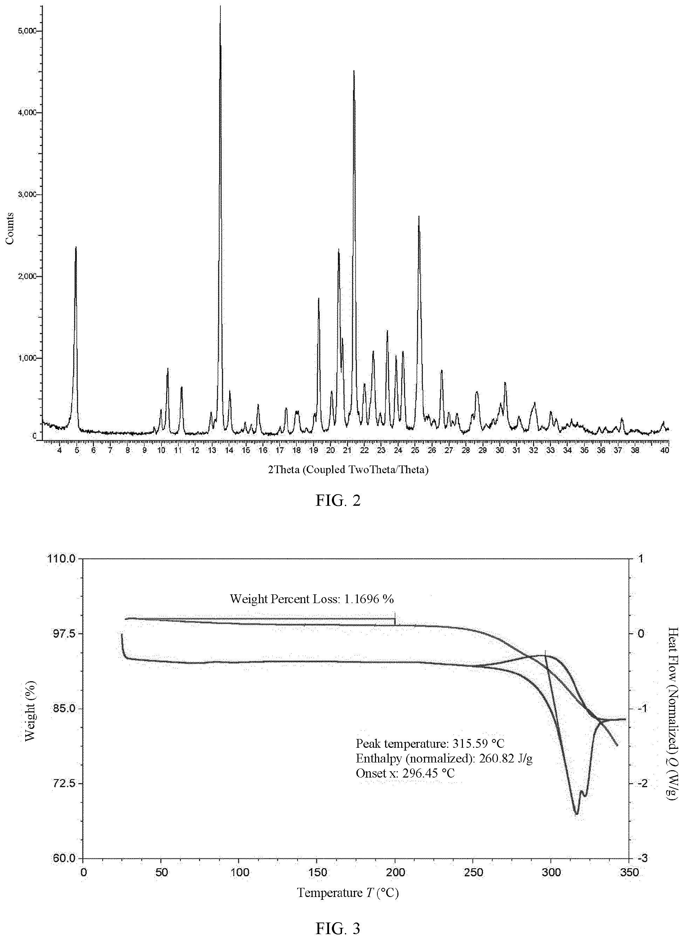

According to an embodiment of the present disclosure, the crystal form I of the dihydrochloride salt of the compound of formula (I) has an X-ray powder diffraction pattern (XRPD) substantially as shown in FIG. 1 or FIG. 2.

In some embodiments, the crystal form I of the dihydrochloride salt of the compound of formula (I) has X-ray powder diffraction analysis data substantially as shown in Table 2 or 3.

According to an embodiment of the present disclosure, the crystal form I of the dihydrochloride salt of the compound of formula (I) has a thermogravimetric analysis curve with a weight loss of 1.17% from 25 °C to 200 °C.

According to an embodiment of the present disclosure, the crystal form I of the dihydrochloride salt of the compound of formula (I) has a differential scanning calorimetry profile showing that it starts to melt and decompose at 285-300 °C.

According to an embodiment of the present disclosure, the crystal form I of the dihydrochloride salt of the compound of formula (I) has a TGA-DSC profile substantially as shown in FIG. 3.

The present disclosure further provides a preparation method for the dihydrochloride salt of the compound of formula (I) described above, comprising the following steps:
adding a compound of formula (I) and a solvent to a reaction flask, slowly adding hydrochloric acid, stirring, separating the obtained solid, and drying to obtain the dihydrochloride salt of the compound of formula (I), wherein hydrochloric acid and the compound of formula (I) are in a molar ratio of (1.5-3):1.

According to an embodiment of the present disclosure, hydrochloric acid and the compound of formula (I) are in a molar ratio of (1.8-2.5):1, such as 2:1.

According to an embodiment of the present disclosure, the solvent is selected from any one of the following solvent groups: water, dimethyl sulfoxide, N-methylpyrrolidone, N,N-dimethylformamide, methanol, tetrahydrofuran-water (V:V = 19:1), and tetrahydrofuran.

In some embodiments, when the solvent is selected from dimethyl sulfoxide or tetrahydrofuran-water (V:V = 19:1), the preparation method further comprises a step of adding an anti-solvent, wherein the anti-solvent is preferably methyl *tert*-butyl ether.

The present disclosure further provides a preparation method for the crystal form I of the dihydrochloride salt of the compound of formula (I) described above, comprising the following steps: adding a compound of formula (I) and a solvent to a reaction flask, and slowly adding hydrochloric acid; optionally, adding a crystal seed of the crystal form I; stirring for crystallization, separating, and drying to obtain the crystal form I of the dihydrochloride salt of the compound of formula (I), wherein hydrochloric acid and the compound of formula (I) are in a molar ratio of (1.5-3):1; the solvent is selected from any one of the following solvent groups: water, dimethyl sulfoxide, N-methylpyrrolidone, N,N-dimethylformamide, methanol, and tetrahydrofuran-water (V:V = 19:1).

According to an embodiment of the present disclosure, when the solvent is selected from dimethyl sulfoxide or tetrahydrofuran-water (V:V = 19:1), the preparation method further comprises a step of adding an anti-solvent, wherein the anti-solvent is preferably methyl *tert*-butyl ether; preferably, dimethyl sulfoxide and methyl *tert*-butyl ether are in a volume ratio of (20-30):8, and more preferably 25:8; preferably, tetrahydrofuran-water (V:V = 19:1) and methyl tert-butyl ether are in a volume ratio of (45-55):8, and more preferably 50:8.

According to an embodiment of the present disclosure, hydrochloric acid and the compound of formula (I) are in a molar ratio of (1.8-2.5):1, such as 2:1.

According to an embodiment of the present disclosure, the crystallization is performed at a temperature of 10-35 °C, preferably 20-30 °C.

According to an embodiment of the present disclosure, the crystallization is performed for 1-72 h, preferably 3-48 h, and more preferably 5-24 h.

According to an embodiment of the present disclosure, the step of separating includes separation of the obtained precipitate from the crystallization liquid by a suitable method such as filtration, centrifugation, or the like.

According to an embodiment of the present disclosure, the method for drying may adopt any suitable known method, preferably drying under reduced pressure (in vacuum). Specific drying conditions are, for example, the temperature is preferably 20-70 °C, and more preferably 30-45 °C; the pressure is preferably a vacuum degree > 0.090 MPa; the drying time is preferably 10-50 h, and more preferably 20-40 h. No matter what drying means is adopted, it is appropriate that the residual amount of the solvent in the obtained product meets the quality standard.

The compound of formula (I) described herein can be prepared by methods known in the art, e.g., as disclosed in the PCT patent document WO2017/140269A1.

The present disclosure further provides a pharmaceutical composition, comprising the dihydrochloride salt of the compound of formula (I) described above or the crystal form I of the dihydrochloride salt of the compound of formula (I) described above, and a pharmaceutically acceptable carrier.

The present disclosure further provides use of the dihydrochloride salt of the compound of formula (I) or the crystal form I thereof described above, or the pharmaceutical composition described above for the manufacture of a medicament for preventing and/or treating the following diseases, wherein the disease is a disease associated with the activity or expression of FGFR, KDR and/or CSF-1R; preferably, the associated disease is a tumor-associated disease, and the tumor-associated disease is selected from the group consisting of: breast cancer, lung cancer, non-small cell lung cancer, squamous lung carcinoma, bladder cancer, urinary tract transitional cell carcinoma, gastric cancer (including gastroesophageal junction adenocarcinoma), pancreatic cancer, prostate cancer, colorectal cancer, myeloma, multiple myeloma, acute myeloid leukemia, liver cancer, melanoma, thyroid cancer liver cancer, head and neck cancer, renal cell carcinoma, glioblastoma, testicular cancer, and/or cholangiocarcinoma.

The medicament is administered to a "subject" or "patient" in an effective dose. The "subject" and "patient" include all members of the animal kingdom, including but not limited to, mammals (e.g., mice, rats, cats, monkeys, dogs, horses, pigs, etc.) and humans.

The present disclosure further provides the dihydrochloride salt of the compound of formula (I) or the crystal form I thereof described above, or the pharmaceutical composition described above for use in treating the following diseases, wherein the disease is a disease associated with the activity or expression of FGFR, KDR and/or CSF-1R; preferably, the associated disease is a tumor-associated disease, and the tumor-associated disease is selected from the group consisting of: breast cancer, lung cancer, non-small cell lung cancer, squamous lung carcinoma, bladder cancer, urinary tract transitional cell carcinoma, gastric cancer (including gastroesophageal junction adenocarcinoma), pancreatic cancer, prostate cancer, colorectal cancer, myeloma, multiple myeloma, acute myeloid leukemia, liver cancer, melanoma, thyroid cancer liver cancer, head and neck cancer, renal cell carcinoma, glioblastoma, testicular cancer, and/or cholangiocarcinoma.

The present disclosure further provides a method for preventing and/or treating a disease associated with the activity or expression of FGFR, KDR and/or CSF-1R, comprising administering to a subject or patient in need thereof a therapeutically effective amount of a dihydrochloride salt of a compound of formula (I) or a crystal form I thereof, or a pharmaceutical composition comprising the dihydrochloride salt of the compound of formula (I) described above or the crystal form I of the dihydrochloride salt of the compound of formula (I) described above. Herein, the disease associated with the activity or expression of FGFR, KDR and/or CSF-1R is a tumor-associated disease, and the tumor-associated disease may be selected from the group consisting of: breast cancer, lung cancer, non-small cell lung cancer, squamous lung carcinoma, bladder cancer, urinary tract transitional cell carcinoma, gastric cancer (including gastroesophageal junction adenocarcinoma), pancreatic cancer, prostate cancer, colorectal cancer, myeloma, multiple myeloma, acute myeloid leukemia, liver cancer, melanoma, thyroid cancer liver cancer, head and neck cancer, renal cell carcinoma, glioblastoma, testicular cancer, and/or cholangiocarcinoma.

### Definitions and Description

Unless otherwise stated, the following terms and phrases used herein are intended to have the following meanings. A particular phrase or term, unless otherwise specifically defined, should not be considered as uncertain or unclear, but construed according to its common meaning.

The dihydrochloride salt of the compound of formula (I) in a solid form mentioned in the present application includes a crystalline form and an amorphous form, etc. of the dihydrochloride salt of the compound of formula (I).

The dihydrochloride salt of the compound of formula (I) in the crystalline form mentioned in the present application includes an anhydrous and non-aqueous solvent-free form, a hydrate form, a non-aqueous solvate form, and a cocrystal form of the dihydrochloride salt of the compound of formula (I).

The "room temperature" is the room temperature in the conventional sense in the field, and is generally 10-30 °C, preferably 25 °C±5 °C.

In the X-ray powder diffraction pattern, the term "substantially" or "substantially as shown in the figure" means that for a certain crystal form that is substantially pure, at least 50%, or at least 60%, or at least 70%, or at least 80%, or at least 90%, or at least 95%, or at least 96%, or at least 97%, or at least 98%, or at least 99% of the peaks in the X-ray powder diffraction pattern appear in the given pattern. Further, when the content of a certain crystal form in the product is gradually reduced, some diffraction peaks attributed to the crystal form in the X-ray powder diffraction pattern may be reduced due to the detection sensitivity of the instrument. In addition, there may be slight errors in the peak positions for any given crystal form, as is also well known in the field of crystallography. For example, the peak positions may shift due to temperature changes, sample movement, or calibration of the instrument, etc. when analyzing a sample, and the error in the measurement of 2θ value is sometimes about ±0.3°, and usually about ±0.2°. Therefore, this error should be taken into account when determining the structure of each crystal form. The terms "substantially" or "substantially as shown in the figure" are also intended to encompass such errors in diffraction peak positions, meaning ±0.3°, preferably ±0.2°.

The term "substantially" or "substantially as shown in the figure" in a DSC profile or TGA profile means that for the same crystal form of the same compound, the error in the thermal transition onset temperature, endothermic peak temperature, exothermic peak temperature, melting point, weight loss onset temperature or weight loss endpoint temperature, etc., in continuous analyses is typically within about 5 °C, generally within about 3 °C. When a certain compound or a crystal form thereof is described as having a certain given thermal transition onset temperature, endothermic peak temperature, exothermic peak temperature, melting point, weight loss onset temperature or weight loss endpoint temperature, etc., it refers to this temperature±5 °C.

The term "tumor" includes benign tumors, malignant tumors, and borderline tumors, wherein malignant tumors are also collectively referred to as cancers.

The term "preventing" as used herein refers to a compound or drug that, when used for treating a disease or condition (e.g., cancer), can reduce the frequency of or delay the onset of symptoms of a medical condition in a subject as compared with a subject who has not been administered the compound or drug (e.g., a pharmaceutical composition product as claimed herein).

The term "treating" as used herein refers to alleviating, relieving, or ameliorating a symptom of a disease or condition, ameliorating an underlying metabolic-induced symptom, and inhibiting a disease or symptom, e.g., arresting the progression of a disease or condition, relieving a disease or condition, causing regression of a disease or condition, relieving a disorder caused by a disease or condition, or arresting a symptom of a disease or condition.

The term "pharmaceutically acceptable carrier" refers to those carriers or auxiliary materials that do not have a significant irritating effect on organisms and do not impair the bioactivity and performance of the active compound.

All solvents used in the present disclosure are commercially available and can be used without further purification.

### Beneficial Effects

The present disclosure provides a dihydrochloride salt of a compound of formula (I), particularly crystal form I. The inventors found that the dihydrochloride salt, particularly the crystal form I, is easy to dry and has good solubility, stable quality, stable thermodynamics, and desirable druggability.

### BRIEF DESCRIPTION OF THE DRAWINGS

FIG. 1 is an XRPD pattern of the crystal form I of the dihydrochloride salt of the compound of formula (I) obtained in Example 2.
FIG. 2 is an XRPD pattern of the crystal form I of the dihydrochloride salt of the compound of formula (I) obtained in Example 3.
FIG. 3 is a TGA-DSC profile of the crystal form I of the dihydrochloride salt of the compound of formula (I) obtained in Example 3.
FIG. 4 is an XRPD comparison diagram of the crystal form I of the dihydrochloride salt of the compound of formula (I) obtained in Example 3 before and after grinding.

### DETAILED DESCRIPTION

The technical solutions of the present disclosure will be further described in detail with reference to the following specific examples. The following examples are merely exemplary illustration and explanation of the present disclosure, and should not be construed as limiting the protection scope of the present disclosure. All techniques implemented based on the contents described above of the present disclosure are encompassed within the protection scope of the present disclosure.

Unless otherwise stated, the starting materials and reagents used in the following examples are all commercially available products or can be prepared by using known methods.

In the following examples, the detection conditions for X-ray powder diffraction (XRPD), thermogravimetric analysis (TGA), differential scanning calorimetry (DSC), and dynamic vapor sorption/desorption (DVS) analysis are as follows:
1. Detection conditions for X-ray powder diffraction (XRPD)
Instrument: BRUKER D8 Advance X-ray powder diffractometer, Germany.
Conditions: Cu-Kα radiation (λ = 1.5418 Å); tube voltage: 40 kV; tube current: 40 mA; 2θ scanning range: 3-40°; scanning step size: 0.02° (2θ); scanning speed: 10 s/step; sample tray: a zero background sample tray.
Methods: a sample is spread on a zero background monocrystalline silicon sample tray and is lightly pressed by a medicine spoon to be flat for measurement.

2. Detection conditions for thermogravimetric analysis (TGA)
Instrument: Discovery TGA 55.
Conditions: temperature range: room temperature to 350 °C; heating rate: 10 °C/min; purging gas: nitrogen; flow rate in equilibration chamber: 40 mL/min; flow rate in sample chamber: 25 mL/min. Methods: 1-5 mg of a sample is placed in a tared open aluminum sample tray, and the amount of the sample is automatically measured in a TGA furnace. The sample is heated to the final temperature at a rate of 10 °C/min and analyzed using data analysis software TRIOS.

3. Differential scanning calorimetry (DSC) analysis
Instrument: Discovery DSC 250.
Conditions: temperature range: 25-350 °C; heating rate: 10 °C/min; purging gas: nitrogen; flow rate: 50 mL/min.
Methods: 1-3 mg of a sample is weighed accurately and placed in an aluminum sample tray with a prick, and the exact mass of the sample is recorded. The sample is analyzed using data analysis software TRIOS.

4. Dynamic vapor sorption/desorption (DVS) analysis
Instrument: DVS Intrinsic plus (SMS, UK).
Methods: about 30 mg of a sample is placed in a tared sample basket, the weight of the sample is automatically measured, and the sample is analyzed according to the parameters in Table 1 below.

**Table 1. Parameters of dynamic vapor sorption/desorption (DVS) analysis**

| Instrument information | SMS, DVS Intrinsic PLUS |
|---|---|
| dm/dt | 0.002%/min |
| Sample amount | 21.5 mg |
| Drying/test temperature | 40°C/25 °C |
| Cycle | Full cycle |
| dm/dt minimum equilibration time | 30 min |
| dm/dt maximum equilibration time | 120 min |
| Data storage rate | 5 s |
| Gas and flow rate | N₂, 200 sccm |
| Post-run flow rate | 200 sccm |
| Step size | 10% RH |
| Method | Sorption: 0, 10, 20, 30, 40, 50, 60, 70, 80, 90 |
| | Desorption: 80, 70, 60, 50, 40, 30, 20, 10, 0 |

### Example 1: High-Throughput Screening of Salt Formation of Compound of Formula (I)

12 acids were selected for high-throughput screening experiments of salt formation according to the pKa of the compound of formula (I): sulfuric acid, phosphoric acid, maleic acid, glutamic acid, tartaric acid, fumaric acid, citric acid, malic acid, ascorbic acid, succinic acid, adipic acid, and hydrochloric acid, and the following 8 solvent systems were selected: A: water; B: water/acetonitrile in a volume ratio of 1:1; C: water/methanol in a volume ratio of 1:1; D: water/acetone in a volume ratio of 1:1; E: water/methanol in a volume ratio of 1:9; F: water/acetone in a volume ratio of 1:9; G: methanol; and H: acetone.

### Experimental procedures:

The compound of formula (I) was dissolved and added to a 96-well plate, and the volume of counterions added was determined according to the molar amount of the compound of formula (I) added and the number of counterion functional groups. The heating time and temperature could be varied as appropriate (generally 40 °C for 1 h). In order to ensure a certain pressure in the flask during the reaction, absolute sealing of a sample needed to be ensured in the processes of uniform mixing, vortexing, and heating, and at least an inner pad made of silicone resin was required throughout the process.

The specific steps are as follows:
1) preparing 0.02 mol/L solutions of acids (except glutamic acid) in tetrahydrofuran-methanol (V:V = 1:1), glutamic acid being an aqueous solution;
2) preparing a 0.01 mol/L solution of the compound of formula (I) in tetrahydrofuran-methanol (V:V = 1:1);
3) adding 1 mL of hydrochloric acid and 0.5 mL of other acids, and then separately adding 1 mL of the compound of formula (I);
4) adding 0.5 mL of each acid, and then separately adding 0.5 mL of the compound of formula (I);
5) after vortexing, reacting in an oven at 40 °C for 1 h, evaporating the organic solvent at room temperature, and finally drying under reduced pressure at 50 °C.

The experimental results show that the compound of formula (I) cannot form salts with glutamic acid and ascorbic acid, and can form salts with other acids to obtain solids, wherein the salts in a relatively good solid state are the dihydrochloride salt, phosphate salt, maleate salt, tartrate salt and fumarate salt.

### Example 2: Crystal Form I of Dihydrochloride Salt of Compound of Formula (I) and Preparation Thereof

A compound of formula (I) (0.52 g, prepared by referring to Preparation Example 10 in WO2017/140269A1) and 25 mL of methanol were added to a reaction flask and stirred. When the system was dispersed uniformly, a solution of hydrochloric acid in methanol (1.2 mL, 2 mol/L) was added dropwise under stirring. The resulting mixture was stirred homogeneously for crystallization for 5 h. The obtained solid was filtered under vacuum, dried under vacuum at 30 °C for 12 h to constant weight, and dried under vacuum at 45 °C until the solvent residue was qualified to obtain a crystal (0.53 g).

The ion chromatography results show that the content of chloride ions in the obtained crystal sample was 11.7%, and the theoretical content of chloride ions in the dihydrochloride salt of the compound of formula (I) was 12.2%, indicating that the obtained crystal is the dihydrochloride salt. The crystal was named "crystal form I". The XRPD characterization pattern of the crystal is shown in FIG. 1, and the characterization data are shown in Table 2.

**Table 2. XRPD characterization data on crystal form I sample in Example 2**

| **2θ (°)** | **Relative peak intensity (%)** | **2θ (°)** | **Relative peak intensity (%)** |
|---|---|---|---|
| 4.964 | 100.0 | 21.394 | 97.3 |
| 9.960 | 7.4 | 21.943 | 10.6 |
| 10.371 | 17.1 | 22.460 | 20.3 |
| 11.192 | 14.0 | 23.340 | 22.2 |
| 13.485 | 93.9 | 23.857 | 13.0 |
| 14.015 | 11.0 | 24.246 | 20.4 |
| 15.704 | 5.5 | 25.179 | 55.9 |
| 17.326 | 4.3 | 26.555 | 12.4 |
| 18.012 | 7.8 | 28.539 | 14.8 |
| 19.318 | 24.9 | 30.222 | 9.3 |
| 20.048 | 8.5 | 31.974 | 5.8 |
| 20.460 | 43.5 | 33.014 | 6.0 |

### Example 3: Crystal Form I of Dihydrochloride Salt of Compound of Formula (I) and Preparation Thereof

A compound of formula (I) (1.0432 g, prepared by referring to Preparation Example 10 in WO2017/140269A1) and 3 mL of purified water were added to a reaction flask and stirred. When the system was dispersed uniformly, 0.35 mL of 2 N hydrochloric acid prepared from 37% concentrated hydrochloric acid and 1.8 mL of pure water was added dropwise. The resulting mixture was stirred until the mixture was completely dissolved. 0.01 g of the crystal form I sample obtained in Example 2 was added at 20-30 °C as a crystal seed, and the resulting mixture was stirred for crystallization for 20-24 h. The obtained solid was filtered under vacuum, dried under vacuum at 30 °C for 12 h to constant weight, and dried under vacuum at 45 °C until the solvent residue was qualified to obtain crystal form I (1.112 g).

The obtained crystal form I exhibited good crystallinity. The XRPD characterization pattern is shown in FIG. 2, and the characterization data are shown in Table 3. The TGA-DSC profile is shown in FIG. 3, and the TGA test results show that the crystal form I sample does not contain water of crystallization or non-aqueous crystallization solvent.

**Table 3. XRPD characterization data on crystal form I sample in Example 3**

| **2θ (°)** | **Relative peak intensity (%)** | **2θ (°)** | **Relative peak intensity (%)** |
|---|---|---|---|
| 4.955 | 37.7 | 21.397 | 87.5 |
| 9.982 | 5.20 | 22.015 | 10.2 |
| 10.384 | 13.7 | 22.526 | 17.7 |
| 11.212 | 10.4 | 23.367 | 22.8 |
| 13.503 | 100 | 23.884 | 17 |
| 14.056 | 9.9 | 24.298 | 17.7 |
| 15.733 | 7 | 25.246 | 50.5 |
| 17.382 | 5.6 | 26.572 | 14.9 |
| 18.029 | 4.4 | 28.654 | 8.4 |
| 19.305 | 31.6 | 30.056 | 6.5 |
| 20.075 | 8.5 | 32.071 | 6.7 |
| 20.496 | 41.1 | 33.032 | 4.8 |

### Example 4: Preparation of Crystal Form I of Dihydrochloride Salt of Compound of Formula (I) under Different Solvent Conditions

Referring to the preparation method in Example 3, the preparation of the crystal form I of the dihydrochloride salt of the compound of formula (I) was performed using the following solvent systems. The results are shown in Table 4 below.

**Table 4. Preparation of crystal form I of dihydrochloride salt of compound of formula (I) under different solvent conditions**

| **Example** | **Solvent** | **Volume (mL)** | **Anti-solvent** | **Volume (mL)** | **Solvent:anti-solvent (V:V)** | **XRPD result** |
|---|---|---|---|---|---|---|
| 4-1 | Water | 4 | / | / | / | Crystal form I |
| 4-2 | Dimethyl sulfoxide | 26 | Methyl *tert-*butyl ether | 8.3 | 25:8 | Crystal form I |
| 4-3 | N-Methylpyrrolidone | 26 | / | / | / | Crystal form I |
| 4-4 | N,N-Dimethylformamide | 52 | / | / | / | Crystal form I |
| 4-5 | Methanol | 52 | / | / | / | Crystal form I |
| 4-6 | Tetrahydrofuran:water (V:V = 19:1) | 52 | Methyl *tert-*butyl ether | 8.3 | 50:8 | Crystal form I |

### Example 5: Preparation of Amorphous Form of Dihydrochloride Salt of Compound of Formula (I)

Referring to the preparation method in Example 3, the preparation of the dihydrochloride salt of the compound of formula (I) was performed using the following solvent system to obtain an amorphous product. The result is shown in Table 5 below.

**Table 5. Preparation result of amorphous form of dihydrochloride salt of compound of formula (I)**

| **Example** | **Solvent** | **Volume (mL)** | **XRPD result** |
|---|---|---|---|
| 5 | Tetrahydrofuran | 52 | Amorphous form |

### Example 6: Preparation of Other Crystal Forms of Hydrochloride Salt of Compound of Formula (I)

Referring to the preparation method in Example 3, the preparation of the hydrochloride salt of the compound of formula (I) was performed using the following solvent systems. The results are shown in Table 6 below.

**Table 6. Preparation results of other crystal forms of dihydrochloride salt of compound of formula (I)**

| **Example** | **Solvent** | **Volume (mL)** | **Anti-solvent** | **Volume (mL)** | **Solvent: anti-solvent V:V** | **XRPD result** |
|---|---|---|---|---|---|---|
| 6-1 | Dichloromethane | 52 | / | / | / | Adhesive substance |
| 6-2 | Ethanol:water (19:1) | 52 | / | / | / | Crystal form I + form 2 (Monohydrochloride salt) |
| 6-3 | Dichloromethane: methanol (V:V = 1:1) | 52 | Methyl *tert*-butyl ether | 2.1 | 50:2 | Crystal form I + form 2 (Monohydrochloride salt) |
| 6-4 | Methanol:water (V: V = 19:1) | 52 | Methyl *tert*-butyl ether | 2.1 | 50:2 | Crystal form I + form 2 (Monohydrochloride salt) |

### Comparative Example 1: Preparation of Other Salts of Compound of Formula (I)

To a 25-mL flask containing 5 mL of a 0.02 mol/mL acid (phosphoric acid, maleic acid, tartaric acid, and fumaric acid) in tetrahydrofuran-methanol (in a volume ratio of 1:1) was separately added 10 mL of a 0.01 mol/mL solution of the compound of formula (I) in tetrahydrofuran-methanol (in a volume ratio of 1:1). The resulting mixtures were mixed uniformly by vortexing. The obtained 4 solutions described above were left to stand at 40 °C for 1 h. The reaction liquids were volatilized and dried at 50 °C for 3-4 h to obtain the phosphate salt, maleate salt, tartrate salt, and fumarate salt of the compound of formula (I).

### Test Example 1: Solubility Experiments of Compound of Formula (I) and Salts Thereof

The solubility of the compound of formula (I) (prepared by referring to Preparation Example 10 in WO2017/140269A1) and the samples obtained in Example 3 and Comparative Example 1 in deionized water and a Na₂HPO₄-citric acid buffer (pH 4.6) medium was tested. The experimental results are shown in Table 7.

**Table 7. Solubility experimental results of compound of formula (I) and different salt forms thereof (25 °C, mg/mL)**

| **Solvent/salt** | **Compound of formula (I)** | **Dihydrochloride salt crystal form I** | **Phosphate salt** | **Maleate salt** | **Tartrate salt** | **Fumarate salt** |
|---|---|---|---|---|---|---|
| Deionized water | 0.008 | 16.2 | 3.5 | 2.0 | 1.4 | 1.3 |
| Na₂HPO₄-citric acid buffer (pH 4.6) | 0.045 | 0.1 | 0.06 | 0.03 | 0.1 | 0.03 |

### Test Example 2: Water Sorption and Desorption Experiments

The samples obtained in Example 3 and Comparative Example 1 were investigated for sorption and desorption of moisture with 40%-80% relative humidity at 25 °C using a dynamic vapor sorption (DVS) instrument to determine the hygroscopicity of various salt forms. The experimental results are shown in Table 8.

**Table 8. Hygroscopicity experimental results of different salt forms of compound of formula (I) (DVS, 40%-80%)**

| **Salt form** | **Dihydrochloride salt crystal form I** | **Phosphate salt** | **Maleate salt** | **Tartrate salt** | **Fumarate salt** |
|---|---|---|---|---|---|
| Hygroscopicit y (%) | 0.65 | 3.75 | 0.81 | 2.86 | 2.11 |

### Test Example 3: Solid Stability Study of Dihydrochloride Salt Crystal Form I

The dihydrochloride salt crystal form I sample obtained in Example 3 was investigated for physical and chemical stability at 40 °C/75% RH (uncovered) and 60 °C (in a covered sample flask) for 7 days, and for physical stability at room temperature/92.5% RH (uncovered) for 10 days. The experimental results are shown in Table 9 below. The results show that the purity and the crystal form of the crystal form I were not significantly changed after the crystal form I was left to stand under the conditions of 40 °C/75% RH (uncovered) and 60 °C (in the covered sample flask) for 7 days; the crystal form of the crystal form I was not significantly changed after the crystal form I was left to stand under the condition of 92.5% RH (uncovered) for 10 days, indicating that the physical and chemical properties of the crystal form I are stable under the test conditions.

**Table 9. Solid stability experimental results**

| **Starting material** | **Purity** | | | **XRPD** | | |
|---|---|---|---|---|---|---|
| | **T0** | **40 °C/75% RH for 7 days** | **60 °C for 7 days** | **40 °C/75% RH for 7 days** | **60 °C for 7 days** | **92.5% RH for 10 days** |
| Crystal form I | 99.85 | 99.86 | 99.85 | Not changed | Not changed | Not changed |

### Test Example 4: Solid Grinding Stability Experiments

### (1) Dropwise addition of solvents and grinding

The following solvents were separately added dropwise to the sample obtained in Example 3 (crystal form I), and the mixtures were ground. The experimental results in Table 10 show that the crystal form was not changed.

**Table 10. Solvent grinding experimental results**

| **#** | **Solvent** | **Result** |
|---|---|---|
| 1 | Methanol | Crystal form I |
| 2 | Ethanol | Crystal form I |
| 3 | Isopropanol | Crystal form I |
| 4 | Acetone | Crystal form I |
| 5 | Butanone | Crystal form I |
| 6 | Acetonitrile | Crystal form I |
| 7 | Ethyl acetate | Crystal form I |

### (2) Solid grinding

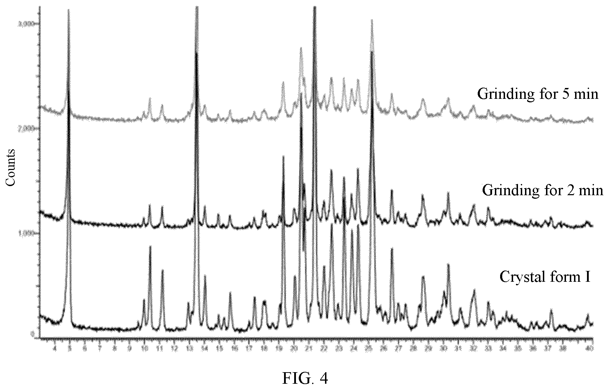

20 mg of the sample in Example 3 was weighed out, added to a mortar, and ground for 2 min and 5 min, respectively. Then, the sample was characterized by XRPD. The experimental results show that the crystal form of the ground sample was not changed, see FIG. 4.

### Test Example 5: Efficacy Experiment of Dihydrochloride Salt of Compound of Formula (I) for Human Lung Cancer NCI-H1581 Nude Mouse Subcutaneous Xenograft Tumor Model

1) Experimental drug: the crystal form I sample of the dihydrochloride salt of the compound of formula (I) obtained in Example 3.
2) Cell strain

The human lung cancer NCI-H1581 cell strain (preserved by Shanghai Institute of Materia Medica, Chinese Academy of Sciences) was inoculated subcutaneously in the right axilla of nude mice with an inoculation amount of the cells of 5 × 10⁶ cells/mouse. The formed xenograft tumor was directly inoculated for use.

### 3) Experimental procedures

The tumor tissue in the vigorous growth phase was cut into about 1.5 mm³ and was inoculated subcutaneously in the right axilla of the nude mouse under sterile conditions. The diameter of the subcutaneous xenograft tumor in the nude mouse was measured using a vernier caliper, and the animals were randomly grouped after the tumors grew to an average volume of about 150-160 mm³. Animals in the Example 3 sample group were orally administered at 10 mg/kg, 5 mg/kg, and 2.5 mg/kg once a day for two consecutive weeks. Animals in the solvent control group (AZD4547) were orally administered at 12.5 mg/kg once a day for two consecutive weeks. Throughout the experiment, the diameter of the xenograft tumor was measured 2 times a week, and the body weight of the mice was measured at the same time. The tumor volume (TV) was calculated as follows: TV = 1/2 × a × b², wherein a and b represent the length and width, respectively. The relative tumor volume (RTV) was calculated from the measurement results using the following formula: RTV = Vt/V₀, wherein V₀ is the tumor volume measured at the time of grouping and administration (d₀), and Vt is the tumor volume at each measurement. The evaluation index of the anti-tumor activity was the relative tumor proliferation rate T/C (%), which was calculated as follows: T/C (%) = (TRTV/CRTV) × 100%, wherein TRTV represents RTV of the treatment group; CRTV represents RTV of the negative control group.

### 4) Results

The experimental results are shown in Table 11. The sample in Example 3 was orally administered at 10 mg/kg, 5 mg/kg, and 2.5 mg/kg once a day for two consecutive weeks, and showed a significant inhibitory effect on the growth of the human lung cancer NCI-H1581 nude mouse subcutaneous xenograft tumors. During the experiment, the animals in each group were in good condition and no mice died.

**Table 11. Effect of sample in Example 3 on proliferation rate of human lung cancer NCI-H1581 nude mouse subcutaneous xenograft tumor model**

| **Group** | **Dose mg/kg** | T/C |
|---|---|---|
| AZD4547 | 12.5 | 20.01 |
| Example 3 | 10 | 17.17 |
| Example 3 | 5 | 33.22 |
| Example 3 | 2.5 | 49.06 |

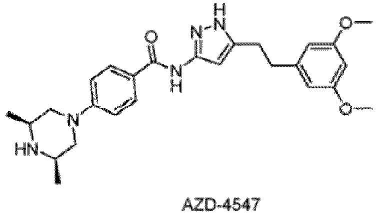

In conclusion, the dihydrochloride salt of the compound of formula (I) of the present disclosure, particularly the crystal form I, has good solubility, low hygroscopicity, stable quality, a stable crystal form, and desirable druggability.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A dihydrochloride salt of a compound of formula (I),

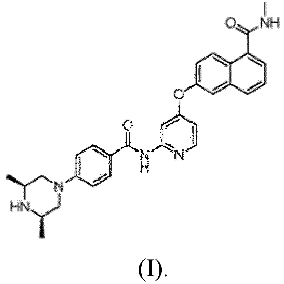

2. The dihydrochloride salt of the compound of formula (I) according to claim 1, wherein the dihydrochloride salt is in a solid form, preferably in a crystalline form.

3. Crystal form I of a dihydrochloride salt of a compound of formula (I), wherein the crystal form I has characteristic peaks at 2θ angles of 5.0±0.2°, 13.5±0.2°, 19.3±0.2°, 20.5±0.2°, 21.4±0.2°, and 25.2±0.2° by X-ray powder diffraction using Cu-Kα radiation;
preferably, the crystal form I of the dihydrochloride salt of the compound of formula (I) has characteristic peaks at 2θ angles of 5.0±0.2°, 10.4±0.2°, 13.5±0.2°, 19.3±0.2°, 20.5±0.2°, 21.4±0.2°, 23.4±0.2°, and 25.2±0.2° by X-ray powder diffraction using Cu-Kα radiation;
more preferably, the crystal form I of the dihydrochloride salt of the compound of formula (I) has characteristic peaks at 2θ angles of 5.0±0.2°, 10.4±0.2°, 13.5±0.2°, 19.3±0.2°, 20.5±0.2°, 21.4±0.2°, 22.5±0.2°, 23.4±0.2°, 23.9±0.2°, 24.3±0.2°, and 25.2±0.2° by X-ray powder diffraction using Cu-Kα radiation.

4. The crystal form I of the dihydrochloride salt of the compound of formula (I) according to claim 3, wherein the crystal form I has characteristic peaks at 2θ angles of 5.0±0.2°, 10.4±0.2°, 11.2±0.2°, 13.5±0.2°, 19.3±0.2°, 20.5±0.2°, 21.4±0.2°, 22.0±0.2°, 22.5±0.2°, 23.4±0.2°, 23.9±0.2°, 24.3±0.2°, 25.2±0.2°, and 26.6±0.2° by X-ray powder diffraction using Cu-Kα radiation;
preferably, the crystal form I has characteristic peaks at 2θ angles of 5.0±0.2°, 10. 0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, and 30.1±0.2° by X-ray powder diffraction using Cu-Kα radiation.

5. The crystal form I of the dihydrochloride salt of the compound of formula (I) according to claim 3 or 4, wherein the crystal form I has an X-ray powder diffraction pattern (XRPD) substantially as shown in FIG. 1 or FIG. 2;
and/or the crystal form I has a thermogravimetric analysis curve with a weight loss of 1.17% from 25 °C to 200 °C;
and/or the crystal form I has a differential scanning calorimetry profile showing that it starts to melt and decompose at 285-300 °C;
and/or the crystal form I has a TGA-DSC profile substantially as shown in FIG. 3.

6. A preparation method for the crystal form I of the dihydrochloride salt of the compound of formula (I) according to any one of claims 3-5, comprising the following steps: adding a compound of formula (I) and a solvent to a reaction flask, and slowly adding hydrochloric acid; optionally, adding a crystal seed of the crystal form I; stirring for crystallization, separating, and drying to obtain the crystal form I, wherein hydrochloric acid and the compound of formula (I) are in a molar ratio of (1.5-3):1; the solvent is selected from any one of the following solvent groups: water, dimethyl sulfoxide, N-methylpyrrolidone, N,N-dimethylformamide, methanol, and tetrahydrofuran-water (V:V = 19:1).

7. The preparation method for the crystal form I of the dihydrochloride salt of the compound of formula (I) according to claim 6, wherein when the solvent is selected from dimethyl sulfoxide or tetrahydrofuran-water (V:V = 19:1), the preparation method further comprises a step of adding an anti-solvent, wherein the anti-solvent is preferably methyl *tert*-butyl ether; preferably, dimethyl sulfoxide and methyl *tert*-butyl ether are in a volume ratio of (20-30):8, and more preferably 25:8; preferably, tetrahydrofuran-water (V:V = 19:1) and methyl tert-butyl ether are in a volume ratio of (45-55):8, and more preferably 50:8;
preferably, hydrochloric acid and the compound of formula (I) are in a molar ratio of (1.8-2.5): 1;
preferably, the crystallization is performed at a temperature of 10-35 °C, preferably 20-30 °C;
preferably, the crystallization is performed for 1-72 h, preferably 3-48 h, and more preferably 5-24 h.

8. A pharmaceutical composition, comprising the dihydrochloride salt of the compound of formula (I) according to claim 1 or 2 or the crystal form I of the dihydrochloride salt of the compound of formula (I) according to any one of claims 3-5, and a pharmaceutically acceptable carrier thereof.

9. Use of the dihydrochloride salt of the compound of formula (I) according to claim 1 or 2 or the crystal form I of the dihydrochloride salt of the compound of formula (I) according to any one of claims 3-5 for the manufacture of a medicament for treating and/or preventing the following diseases, wherein the disease is a disease associated with the activity or expression of FGFR, KDR and/or CSF-1R.

10. The use according to claim 9, wherein the associated disease is a tumor-associated disease, and the tumor-associated disease is selected from the group consisting of: breast cancer, lung cancer, non-small cell lung cancer, squamous lung carcinoma, bladder cancer, urinary tract transitional cell carcinoma, gastric cancer (including gastroesophageal junction adenocarcinoma), pancreatic cancer, prostate cancer, colorectal cancer, myeloma, multiple myeloma, acute myeloid leukemia, liver cancer, melanoma, thyroid cancer liver cancer, head and neck cancer, renal cell carcinoma, glioblastoma, testicular cancer, and/or cholangiocarcinoma.
